(19) Europäisches Patentamt
European Patent Office
Office européen des brevets

(11) EP 1 657 294 A1

(12) # EUROPÄISCHE PATENTANMELDUNG

(43) Veröffentlichungstag:
**17.05.2006 Patentblatt 2006/20**

(51) Int Cl.:
***C11B 9/00*** *(2006.01)* ***A61K 8/18*** *(2006.01)*
***A61K 8/99*** *(2006.01)*

(21) Anmeldenummer: **04027198.3**

(22) Anmeldetag: **16.11.2004**

(84) Benannte Vertragsstaaten:
**AT BE BG CH CY CZ DE DK EE ES FI FR GB GR HU IE IS IT LI LU MC NL PL PT RO SE SI SK TR**
Benannte Erstreckungsstaaten:
**AL HR LT LV MK YU**

(71) Anmelder: **Symrise GmbH & Co. KG**
**37603 Holzminden (DE)**

(72) Erfinder:
- **Panten, Johannes, Dr.**
  **37671 Höxter (DE)**
- **Bertram, Heinz-Jürgen, Dr.**
  **37603 Holzminden (DE)**

(74) Vertreter: **Eisenführ, Speiser & Partner**
**Patentanwälte Rechtsanwälte**
**Postfach 10 60 78**
**28060 Bremen (DE)**

(54) **Antiallergene Riechstoffzubereitungen**

(57) Die Erfindung betrifft das Gebiet der Riechstoffe, Riechstoffzubereitungen, kosmetischen und/oder dermatologischen Produkte sowie der Fullerene. Die Erfindung zielt auf eine Erhöhung der Lagerstabilität, der Stabilisierung gegen Oxidation und eine Verringerung der allergenen Wirkung von Riechstoffen unter Verwendung von einem Antioxidans, das einen Fulleren-Körper besitzt.

EP 1 657 294 A1

**Beschreibung**

Die Erfindung betrifft das Gebiet der Riechstoffzubereitungen und der kosmetischen und/oder dermatologischen Produkte.

Riechstoffe und Parfümöle spielen eine bedeutende Rolle im täglichen Leben der Menschen. Heute erstreckt sich ihr Anwendungsgebiet auf fast alle Bereiche des menschlichen Lebens, vornehmlich jedoch Körperpflege- und Haushaltsprodukte. Seit kurzem geht man davon aus, dass einige Riechstoffe für allergische Reaktionen der menschlichen Haut ursächlich oder zumindest mitverantwortlich sind. Man vermutet eine Haptenisierung von Riechstoffen durch die Proteine der oberen Hautschicht. Diese "Allergische Kontakt-Dermatitis" durch Riechstoffe fällt in den sog. Allergie-Typ IV, eine durch T-Zellen vermittelte verzögerte Überempfindlichkeitsreaktion. Die Entwicklung dieses Typs läuft in zwei verschiedenen Phasen ab: In der Induktionsphase dringt ein kleines Molekül durch die Corneum-Schicht der Haut in die Epidermis ein und bindet dort an oder reagiert mit Hautproteinen. Dieser Komplex wird durch antigenpräsentierende Zellen der Haut (wie die Langerhans-Zellen) aufgenommen, bearbeitet und zusammen mit Klasse II MHC-Molekülen den T-Lymphozyten präsentiert. Die dadurch aktivierten T-Zellen teilen sich und differenzieren in sensibilisierte T-Zellen und Gedächtniszellen. In der Expressionsphase aktiviert eine folgende Aussetzung desselben Duftstoffes die sensibilisierten T Zellen, und bewirkt die Freisetzung von verschiedenartigen Cytokinen (beispielsweise IL-2, IFN-$\gamma$, MIF, TNF-$\beta$). Die Cytokine verursachen eine Ansammlung und Aktivierung von Makrophagen. Die Makrophagen setzen Enzyme frei, die eine lokalisierte Gewebe-Zerstörung verursachen.

Besonders Riechstoffen mit einer $\alpha$,$\beta$-ungesättigten Carbonylgruppe neigen zu Haptenisierungsreaktionen, insbesondere mit Proteinen, die Aminosäuren mit freien Aminogruppen enthalten, da Riechstoffe leicht über eine nucleophile 1,4-Addition an Aminogruppen binden können. Aber auch solche Riechstoffe, die z.B. durch radikalische Oxidation mit Luftsauerstoff solche ungesättigten Strukturen bilden können, sind häufig allergen. Gewöhnlich werden deshalb bei Einsatz von oxidationsempfindlichen Stoffen Radikalfänger bzw. Antioxidantien zugesetzt. Diese bestehen häufig aus phenolischen Strukturen, die beim Abfangen von Sauerstoffradikalen zu chinoiden Strukturen umgewandelt werden. Diese wiederum stellen selber $\alpha$,$\beta$-ungesättigte Strukturen dar, die in der Lage sind, mit Aminosäuren zu reagieren und damit bei Hautkontakt allergieauslösende Reaktionen hervorzurufen.

Besonders bedeutsame oxidationsempfindliche Riechstoffe, die zu allergieauslösenden Strukturen oxidiert werden können, sind:

Limonen:

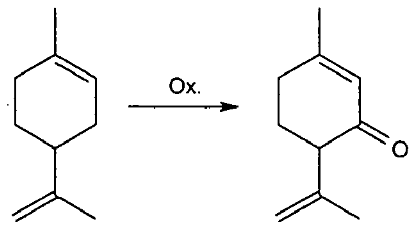


Geraniol:

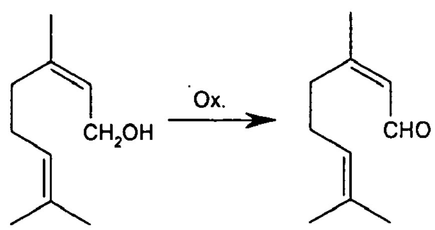


Eugenol:

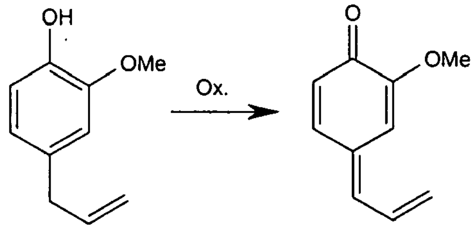

Es war daher eine Aufgabe der vorliegenden Erfindung, insbesondere oxidationsempfindliche Riechstoffe zu stabilisieren und ihre allergene Wirkung zu verringern. Eine weitere Aufgabe war es, entsprechend stabilisierte Riechstoffzubereitungen, kosmetische und/oder dermatologische Produkte anzugeben.

Erfindungsgemäß wird deshalb eine Riechstoffzubereitung angegeben, umfassend einen Riechstoff und ein erstes Antioxidans. Die Riechstoffzubereitung ist dadurch gekennzeichnet, dass sie ferner ein zweites Antioxidans umfasst, wobei das zweite Antioxidans einen Fulleren-Körper besitzt.

Erfindungsgemäß wird unter einem Fulleren-Körper ein Molekül bzw. Molekül-Abschnitt verstanden, das bzw. der Kohlenstoff-Fünf- und/oder Sechsringe enthält, die zusammen einen weiteren Ring bilden, dessen Mittelachse im wesentlichen parallel zur Ringebene der einzelnen Fünf- bzw. Sechsringe verläuft. Fulleren-Körper im Sinne der Erfindung besitzen daher zumindest einen zylinderförmigen Abschnitt, wobei die Fünf- und/oder Sechsringe des Abschnitts den Zylindermantel des Abschnitts bilden. Beispielsweise sind die häufig "Buckyball" genannten $C_{60}$-Fullerene mit 12 Fünf- und 20 Sechsringen Fulleren-Körper im Sinne der Erfindung. Weitere Fulleren-Körper in Sinne der Erfindung sind $C_{70}$ (12 Fünfringe, 25 Sechsringe), $C_{78}$, $C_{82}$, $C_{84}$, $C_{90}$, $C_{94}$ und $C_{96}$. Ferner sind Kohlenstoff-Nanoröhren Fulleren-Körper im erfindungsgemäßen Sinne. Fulleren-Körper im Sinne der Erfindung werden daher häufig einen Durchmesser von 7 bis 15 Å besitzen.

Die Verwendung von Fullerenen in kosmetischen Produkten ist an sich bekannt.

Die EP 770577 B1 und die dort zitierten Referenzen beschreiben den Einsatz von neuartigen Fullerenderivaten mit verbesserten toxikologischen Eigenschaften in diversen medizinischen Anwendungen (u.a. als Vorbeugung gegen Alzheimer und Krebs etc.), in Zigarettenfiltern, um Radikale zu absorbieren und als antiaging Mittel in Skin Care- und anderen kosmetischen Präparaten. Im Unterschied zu den allergieauslösenden Reaktionen entstehen beim antiaging-Prozeß die Peroxide in der Hautschicht durch Oxidation der Lipidschichten mit Sauerstoff.

In der US 6,380,434 und den dort zitierten Referenzen werden weitere Fullerenderivate beschrieben, ohne dass neue Anwendungsgebiete eröffnet würden.

In den Dokumenten JP 05017328 und JP 05017327 werden kosmetische Zubereitungen in Verbindung mit Siliconen und Fullerenen als Inhaltsstoffen beschrieben.

In der japanischen Zeitschrift Bihada-Hifu Bogo to Baiogijutsu (2003), 229-238 werden die antioxidativen und radikal-abfangenden Eigenschaften der Fullerene und Fulleren-Derivate in Verbindung mit Hautschutz erwähnt.

In der ebenfalls japanischen Zeitschrift Fragrance Journal 2003, 31 (8), 40-48 werden die antioxidativen Effekte von Fulleren-Derivaten speziell bei den Peroxylipiden der Keratinocyten in der menschlichen Haut beschrieben.

In der DE 198 07 979 werden dendrimere Fullerenderivate beschrieben, die eine verbesserte Wasserlöslichkeit aufweisen und als Mittel für therapeutische Zwecke eingesetzt werden können.

In allen bisherigen Dokumenten des Standes der Technik wird ausschließlich auf das Phänomen der Hautalterung eingegangen. Die Hautalterung stellt aus medizinischer Sicht jedoch ein von Kontaktallergien klar unterschiedenes Krankheitsbild dar, da die Hautalterung hervorgerufen wird durch die Oxidation der Lipidschichten der Haut, während Riechstoff-Kontaktallergien auf eine Haptenisierungsreaktion mit Hautproteinen zurückgehen.

Gegen den Einsatz von Fullerenen insbesondere in kosmetischen und dermatologischen Produkten hat zudem gesprochen, dass Fullerene in organischen Lösungsmittel eine intensive rötlich-violette Färbung hervorrufen. Die violette Farbe von Fulleren $C_{60}$ wird durch einen symmetrieverbotenen Übergang im sichtbaren Bereich des UVNIS Spektrums verursacht. Starke Absorptionsbanden treten auch bei 211, 256 und 328 nm auf. Die Färbung der Fullerene ist insbesondere in Kosmetikprodukten und Sonneschutzmitteln unerwünscht.

Überraschenderweise wurde nunmehr jedoch gefunden, dass Substanzen mit einem Fulleren-Körper bereits in solchen niedrigen Konzentrationen merklich als Antioxidans wirken, in denen sie noch keine nennenswerte Verfärbung hervorrufen, dazu unten mehr (s. dazu auch die Beispiele). Zweite Antioxidantien mit Fulleren-Körper sind daher besonders vorteilhaft geeignet, Riechstoffe in Riechstoffzubereitungen und kosmetischen und/oder dermatologischen Produkten gegen Oxidation zu stabilisieren, ihre allergene Wirkung zu verringern oder ganz aufzuheben und die Lagerstabilität solcher Riechstoffe, Riechstoffzubereitungen und kosmetischer und/oder dermatologischer Produkte zu erhöhen.

Durch diese Erkenntnis ist es erstmals möglich, herkömmliche Antioxidantien in Riechstoffzubereitungen zumindest teilweise durch Antioxidantien mit Fulleren-Körper zu ersetzen. Beste Ergebnisse wurden erhalten, wenn zweite Antioxidantien mit Fulleren-Körper zusammen mit anderen, ersten Antioxidantien in Riechstoffmischungen eingesetzt wurden. Dabei ist es sinnvoll und bevorzugt, wenn die ersten Antioxidantien solche sind, die keinen Fulleren-Körper besitzen.

Antioxidantien sind im Sinne der Erfindung definiert als Substanzen, die in im Vergleich zu dem oxidierbaren Substrat (Riechstoff) kleinen Konzentrationen die Oxidation des Substrats signifikant verzögern oder gänzlich verhindern.

Einige natürliche und wichtige Antioxidantien sind die Tocopherole (Vitamin E), L-Ascorbinsäure (Vitamin C) und Glutathion, sowie Ubichinol, β-Carotin und Bilirubin (Abbauprodukt von Porphyrin-Derivaten) (vgl. C.-M. Andersson, A. Hallberg, T. Högberg, "Advances in the Development of Pharmaceutical Antioxidants", in Adv. Drug Res., B. Testa, U.A. Meyer (Hrsg.), Academic Press, London, 1996, S. 65-180). Daneben sind noch die mehrwertigen Säuren wie z.B. Zitronensäure und Aminosäuren als Antioxidantien zu erwähnen, die chelatisierend wirken und damit Metallionen maskieren können. Ein weiteres Antioxidans speziell in der Haut ist das in den Melanocyten gebildete Melanin, ein meist braun-schwarzes Polymer, dass durch Oxidation und Polymerisation aus aromatischen Aminosäuren wie L-Tyrosin gebildet wird (vgl. M.R. Chedekel, Cosmetics & Toiletries 1996, 111(1), 71-74).

Die wichtigsten nichtnatürlichen Antioxidantien, die vor allem in der Nahrungsmittelindustrie zur Stabilisierung von Fetten und Ölen eingesetzt werden, sind 2- bzw. 3-tert-Butyl-4-methoxyphenol (1:9-Gemisch, butyliertes Hydroxyanisol, BHA) und 2,6-Di-tert-butyl-4-methylphenol (butyliertes Hydroxytoluol, BHT), Gallussäurepropylester (PG), Gallussäuredodecanylester (DG) und 2-tert-Butyl-1,4-dihydroxybenzol (TBHQ). Einige dieser synthetischen Antioxidantien sind allerdings aus toxikologischer Sicht als bedenklich eingestuft worden (vgl. S.M. Barlow, "Toxicological Aspects of Antioxidants Used as Food Additives", in Food Antioxidants, B.J.F. Hudson (Hrsg.), Elsevier, London, 1990, S. 253-307). So können die oben aufgeführten Antioxidantien bei den erwähnten Redoxprozessen u.a. auch chinoide Strukturen bilden, die aus dermatologischer Sicht zu einem erhöhten Allergierisiko führen können.

Die natürlichen und nicht-natürlichen Riechstoffe können allein oder gemischt vorteilhaft verwendet werden in Kombination mit einem zweiten Antioxidans, das einen Fulleren-Körper besitzt, um einen Riechstoff gegen Oxidation zu stabilisieren und/oder seine allergene Wirkung herabzusetzen und/oder aufzuheben.

Als erste Antioxidantien sind allein oder in Mischung zweier oder mehrerer dieser Antioxidantien besonders geeignet: Aminosäuren (z.B. Glycin, Histidin, 3,4-Dihydroxyphenylalanin, Tyrosin, Tryptophan) und deren Derivate, Imidazole (z.B. Urocaninsäure) und deren Derivate, Peptide (D,L-Carnosin, D-Carnosin, L-Carnosin, Anserin) und deren Derivate, Carotinoide, Carotine (z.B. α-Carotin, β-Carotin, Lycopin) und deren Derivate, Chlorogensäure und deren Derivate, Liponsäure und deren Derivate, Aurothioglucose, Propylthiouracil und andere Thiole (z.B. Thioredoxin, Glutathion, Cystein, Cystin, Cystamin und deren Glycosyl- und N-Acylderivate oder deren Alkylester) sowie deren Salze, Dilaurylthiodipropionat, Distearylthiodipropionat, Thiodipropionsäure und deren Derivate sowie Phenolsäureamide phenolischer Benzylamine (z.B. Homovanillinsäure-, 3,4-Dihydroxyphenylessigsäure-, Ferulasäure-, Sinapinsäure-, Kaffeesäure-, Dihydroferulasäure-, Dihydrokaffeesäure-, Vanillomandelsäure- oder 3,4-Dihydroxymandelsäureamide des 3,4-Dihydroxybenzyl-, 2,3,4-Trihydroxybenzyl- bzw. 3,4,5-Trihydroxybenzylamins), ferner (Metall-)chelatoren (z.B. 2-Hydroxyfettsäuren, Phytinsäure, Lactoferrin), Huminsäure, Gallensäuren, Gallenextrakte, Bilirubin, Biliverdin, Folsäure und deren Derivate, Ubichinon und Ubichinol und deren Derivate, Vitamin C und dessen Derivate (z.B. Ascorbylpalmitat, Magnesiumascorbylphosphat, Ascorbylacetat), Tocopherole und Derivate (z.B. Vitamin-E-Acetat), Vitamin A und Derivate (z.B. Vitamin-A-Palmitat), Rutinsäure und deren Derivate, Flavonoide (z.B. Quercetin, α-Glucosylrutin) und deren Derivate, Phenolsäuren (z.B. Gallussäure, Ferulasäure) und deren Derivate (z.B. Gallussäurepropylester, -ethylester, -octylester), Furfurylidenglucitol, Dibutylhydroxytoluol, Butylhydroxyanisol, Harnsäure und deren Derivate, Mannose und deren Derivate, Zink und dessen Derivate (z.B. ZnO, $ZnSO_4$), Selen und dessen Derivate (z.B. Selenomethionin), Stilbene und deren Derivate (z.B. Stilbenoxid, Resveratrol) und weitere geeignete Derivate dieser genannten Wirkstoffe.

Als Antioxidantien mit Fulleren-Körper sind erfindungsgemäß besonders solche bevorzugt, die einen Fullerenkörper gemäß Formel I umfassen,

$$F(-X)_m(-Y-Z)_n$$

sowie ihre Salze.

F = ein Fullerenkörper;

X = unabhängig voneinander $-OH^-$, $-SH^-$, $-NH_2$, $-COO^-$, $-SO_3^-$, $-PO_3^-$, $-O-PO_3^{2-}$, $-O-PO(O^-)-O-PO_3^{2-}$, $O-PO(O^-)-O-PO(O^-)-O-PO_3^{2-}$, $O-PO(O^-)-O-CH_2-CH_2-NH_3^+$, $NH_3^+$, $N^+H_2R_a$, $N^+HR_aR_b$, oder $N^+R_aR_bR_c$;

Y = A-B;

A = unabhängig voneinander $-CH_2-$, -O-, -S-, -NHCONH-, oder -NHCO-;

B = $R_d-O-[Si(Me)_2-O-]_{1-100}$, $C_{1-2000}$ Alkyl (ein zweiwertiges Radikal), $C_{6-40}$ Aryl (ein zweiwertiges Radikal), $C_{7-60}$ Arylalkyl oder Alkylaryl (ein zweiwertiges Radikal), $(C_{1-30}$ Alkylether oder -thioether$)_{1-100}$, $(C_{6-40}$ Arylether oder -thioether$)_{1-100}$, $(C_{7-60}$ Arylalkylether oder -thioether$)_{1-100}$, $(C_{7-60}$ Alkylarylether oder -thioether$)_{1-100}$, $(C_{2-50}$ Alkylester$)_{1-100}$, $(C_{7-60}$ Arylester$)_{1-100}$, $(C_{8-70}$ Alkylaryl- oder Arylalkylester$)_{1-100}$, R-CO-O-$(C_{1-30}$ Alkylether$)_{1-100}$, R-CO-O-$(C_{6-40}$ Arylether$)_{1-100}$,

R-CO-O-($C_{7-60}$ Arylalkyl oder Alkylarylether)$_{1-100}$, ($C_{4-50}$ Alkylurethan)$_{1-100}$, ($C_{14-60}$ Arylurethan)$_{1-100}$, ($C_{10-80}$ Alkylaryl oder Arylalkylurethan)$_{1-100}$, ($C_{5-50}$ Alkylharnstoff)$_{1-100}$, ($C_{14-60}$ Arylharnstoff)$_{1-100}$, ($C_{10-80}$ Arylalkyl oder Alkylarylharnstoff)$_{1-100}$, ($C_{2-50}$ Alkylamid)$_{1-100}$, ($C_{7-60}$ Arylamid)$_{1-100}$, ($C_{8-70}$ Arylalkyl- oder Alkylarylamid)$_{1-100}$, ($C_{3-30}$ Alkylanhydrid)$_{1-100}$, ($C_{8-50}$ Arylanhydrid)$_{1-100}$, ($C_{9-60}$ Alkylaryl- oder Arylalkylanhydrid)$_{1-100}$, ($C_{2-30}$ Alkylcarbonat)$_{1-100}$, ($C_{7-50}$ Arylcarbonat)$_{1-100}$, ($C_{8-60}$ Arylalkyl- oder Alkylarylcarbonat)$_{1-100}$, $R_1$-O-CO-NH-($R_2$ oder Ar-$R_2$-Ar)-NH-CO-O-($C_{1-30}$ Alkylether, $C_{6-40}$ Arylether, $C_{7-60}$ Arylalkyl-, $C_{7-60}$ Alkylarylether, $C_{2-50}$ Alkylester, $C_{7-60}$ Aryl ester, $C_{8-70}$ Arylalkyl- oder Alkylarylester, $C_{2-50}$ Alkylester, $C_{7-60}$ Arylester, $C_{8-70}$ Alkylaryl- oder Arylalkylester, $C_{1-30}$ Alkylether, $C_{6-40}$ Arylether oder $C_{7-60}$ Alkylaryl- oder Arylalkylether)$_{1-100}$, $R_1$-NH-CO-O-($R_2$ or Ar-$R_2$-Ar)-NH-CO-O-($C_{1-30}$ Alkylether, $C_{6-40}$ Arylether, $C_{7-60}$) Alkylaryl- oder Arylalkylether)$_{1-100}$-CO-NH-($R_2$ oder Ar-$R_2$-Ar)-NH-CO-O, $R_1$-O-CO-NH-($R_2$ oder Ar-$R_2$-Ar)-NH-CO-O-($C_{2-50}$ Alkylester, $C_{7-60}$ Arylester, $C_{8-70}$ Alkylaryl- oder Arylalkylester)$_{1-100}$-$R_3$-O-CO-NH-($R_2$ oder Ar-$R_2$-Ar)-NH-CO-O, $R_1$-NH-CO-NH-($R_2$ oder Ar-$R_2$-Ar)-NH-CO-O-($C_{1-30}$ Alkylether, $C_{6-40}$ Arylether, $C_{7-60}$ Arylalkyl- oder Alkylarylether, $C_{2-50}$ Alkylester, $C_{7-60}$ Arylester oder $C_{8-70}$ Arylalkyl- oder Alkylarylester)$_{1-100}$, $R_1$-NH-CO-NH-($R_2$ oder Ar-$R_2$-Ar)-NH-CO-O-($C_{1-30}$ Alkylether, $C_{6-40}$ Arylether oder $C_{7-60}$ Alkylaryl- oder Arylalkylether)$_{1-100}$-CO-NH-($R_2$ oder Ar-$R_2$-Ar)-NH-CO-O, $R_1$-NH-CO-NH-($R_2$ oder Ar-$R_2$-Ar)-NH-CO-O-($C_{2-50}$ Alkylester, $C_{7-60}$ Arylester oder $C_{8-70}$ Alkylaryl- oder Arylalkylester)$_{1-100}$-$R_3$-O-CO-NH-($R_2$ oder Ar-$R_2$-Ar)-NH-CO-O, $R_{1}$-O-CO-NH-($R_2$ oder Ar-$R_2$-Ar)-NH-CO-NH-($C_{2-50}$ Alkylamid, $C_{7-60}$ Arylamid oder $C_{7-70}$ Arylalkyl- oder $C_{7-70}$ Alkylarylamid)$_{1-100}$, oder $R_1$-NH-CO-NH-($R_2$ oder Ar-$R_2$-Ar)-NH-CO-NH-($C_{2-50}$ Alkylamid, $C_{7-60}$ Arylamid, oder $C_{7-70}$ Arylalkyl- oder Alkylarylamid)$_{1-100}$;

Z = C-D;

C = R, R-Ar, Ar oder Ar-R;

D = unabhängig voneinander $-OH^-$, $-SH^-$, $-NH_2$, $-COO^-$, $-SO_3^-$, $-PO_3^-$, $-O-PO_3^{2-}$, $-O-PO(O^-)-O-PO_3^{2-}$, O-PO(O-)-O-PO$(O^-)$-O-$PO_3^{2-}$, O-PO(O-)-O-$CH_2$-$CH_2$-$NH_3^+$, $NH_3^+$, $N^+H_2R_a$, $N^+HR_aR_b$, oder $N^+R_aR_bR_c$ ;

m, n = 0-30; m+n = 2-30; vorausgesetzt, dass, wenn X = -OH, $-NH_2$, oder $NH_3^+$, $N^+H_2R_a$, $N^+HR_aR_b$ oder $N^+R_aR_bR_c$ Kation, dann ist n nicht 0;

R, $R_1$, $R_2$, $R_3$, $R_a$, $R_b$, $R_c$, $R_d$ = $C_{1-20}$ Alkyl oder Alkylen; und Ar = $C_{6-40}$ Aryl oder Arylen.

Die erfindungsgemäßen Riechstoffzubereitungen enthalten erste und zweite Antioxidansmittel in einer wirksamen Menge, insbesondere zum Stabilisieren des Riechstoffs gegen Oxidation an Luft und/oder zum Verbessern der Lagerungsstabilität und/oder zum Verringern der allergenen Wirkung des Riechstoffs, und gegebenenfalls andere Bestandteile. Die erfindungsgemäßen Riechstoffzubereitungen können dabei als "Wasser in Öl"-, "Öl in Wasser"-, "Wasser in Öl in Wasser"- oder "Öl in Wasser in Öl"-Emulsionen, als Mikroemulsionen, als Gele, als Lösungen z.B. in Ölen, Alkoholen oder Siliconölen vorliegen. Weitere übliche Hilfs- und Zusatzstoffe können in Mengen von 5 bis 99,9999 Gew.-%, vorzugsweise 10 bis 80 Gew.-%, bezogen auf das Gesamtgewicht der Formulierung, enthalten sein. Ferner können die Formulierungen Wasser in einer Menge bis zu 99,999 Gew.-%, vorzugsweise 5 bis 80 Gew.-%, bezogen auf das Gesamtgewicht der Formulierung, aufweisen.

Zur Herstellung der erfindungsgemäßen Zubereitungen können die ersten und zweiten Antioxidantien auch vor dem Mischen mit dem oder den Riechstoff(en) in Liposomen, z.B. ausgehend von Phosphatidylcholin, in Microsphären, in Nanosphären oder auch in Kapseln aus einer geeigneten Matrix, z.B. aus natürlichen oder synthetischen Wachsen, beispielsweise Bienenwachs, Carnaubawachs, Siliconwachs oder Stearylalkohol, Eicosanol, Cetylalkohol, Stearin oder Paraffinwachs oder aus Gelatine, eingearbeitet werden. Eine weitere Ausführungsform besteht darin, dass die zweiten und ersten Antioxidantien vorher mit Komplexbildnern, beispielsweise mit Cyclodextrinen oder Cyclodextrinderivaten, bevorzugt Methylcyclodextrin, komplexiert werden.

Eine bevorzugte erfindungsgemäße Riechstoffzubereitung ist dadurch gekennzeichnet, dass das zweite Antioxidans in einem Anteil von 0,001 bis 3 Gew.-% bezogen auf die gesamte Riechstoffzubereitung vorliegt. Bereits in diesen niedrigen Anteilen entfaltet sich die antioxidative Wirkung der erfindungsgemäßen zweiten Antioxidans.

Besonders bevorzugt ist eine Riechstoffzubereitung, die einen Gesamtanteil an ersten und zweiten Antioxidans von 0,1-2 Gew.-% bezogen auf die gesamte Riechstoffzubereitung aufweist. Dementsprechend ist es besonders bevorzugt, wenn der Anteil an zweitem Antioxidans an der Riechstoffmischung 0,01-2 Gew.-%, besonders bevorzugt 0,01-0,2 Gew.-% beträgt. Bereits bei derart geringen Konzentrationen ist eine überraschend ausgeprägte Stabilisierung der Riechstoffmischung und eine überraschend starke Verringerung der allergenen Wirkung des Riechstoffs bzw. der Riechstoffe zu beobachten.

Ferner ist eine solche Riechstoffmischung bevorzugt, bei der der Fulleren-Körper des zweiten Antioxidans ein geschlossener Käfig-Körper ist. Solche zweiten Antioxidantien sind insbesondere die häufig "Buckyball" genannten $C_{60}$-Fullerene mit 12 Fünf- und 20 Sechsringen, aber auch $C_{70}$ (12 Fünfringe, 25 Sechsringe), $C_{78}$, $C_{82}$, $C_{84}$, $C_{90}$, $C_{94}$ und $C_{96}$, wobei diese Fulleren-Körper keine Kugelform, sondern im Wesentlichen ellipsoidale Formen besitzen. Diese Antioxidantien sind besonders leicht in hoher Reinheit erhältlich und beeinflussen insbesondere in den erfindungsgemäß eingesetzten Konzentrationen nicht den Geruchseindruck der erfindungsgemäßen Riechstoffzubereitung.

Besonders bevorzugt wiederum ist eine Riechstoffzubereitung, die einen Riechstoff enthält, der bei Oxidation an Luft eine $\alpha,\beta$-ungesättigte Carbonylfunktion bildet. Derartige Riechstoffe haben ein besonders hohes allergenes Potential. Durch die Verwendung eines zweiten Antioxidans in Kombination mit einem ersten Antioxidans lassen sich

solche Riechstoffzubereitungen jedoch besonders gut gegen Oxidation stabilisieren, so dass die derart stabilisierte Riechstoffmischung eine im Vergleich zu Riechstoffmischungen ohne zweitem Antioxidans verringerte allergene Wirkung besitzt.

Erfindungsgemäß kann ein Antioxidans auch eine Mischung zweier oder mehrerer Antioxidantien sein. Das erste Antioxidans kann also eine Mischung zweier oder mehrerer Antioxidantien ohne Fulleren-Körper sein, während das zweite Antioxidans eine Mischung zweier oder mehrerer Antioxidantien mit Fulleren-Körpern sein kann. Ebenfalls erfindungsgemäß kann die Riechstoffmischung einen oder mehrere Riechstoffe umfassen. Soweit im Rahmen dieser Beschreibung daher die Singular-Form Antioxidans bzw. Riechstoff verwendet wird, ist darunter immer auch eine Mischung zweier oder mehrerer der Antioxidantien bzw. Riechstoffe zu verstehen.

Die erfindungsgemäße Riechstoffmischung ist vorzugsweise enthalten in einem kosmetischen und/oder dermatologischen Produkt; sie vermittelt diesem Produkt die oben beschriebenen Vorteile, insbesondere die verbesserte Stabilität gegen Oxidation an Luft und eine verringerte allergene Wirkung des Riechstoffs.

Die Menge der ersten, herkömmlichen Antioxidantien (eine oder mehrere Verbindungen), die mit den erfindungsgemäßen zweiten Antioxidantien nicht identisch sind, in den erfindungsgemäßen kosmetischen und/oder dermatologischen Produkten bzw. Zubereitungen, beträgt vorzugsweise 0,0001 bis 3 Gew.-%, besonders bevorzugt 0,001 bis 1 Gew.-%, insbesondere bevorzugt 0,001 bis 0,2 Gew.-%, bezogen auf das Gesamtgewicht der Produkte.

Die erfindungsgemäßen kosmetischen und dermatologischen Zubereitungen können kosmetische Hilfs- und Zusatzstoffe enthalten, wie sie üblicherweise in solchen Zubereitungen verwendet werden, z.B. Sonnenschutzmittel (z.B. organische oder anorganische Lichtfiltersubstanzen, bevorzugt Mikropigmente), Konservierungsmittel, Bakterizide, Fungizide, Viruzide, Kühlwirkstoffe, Pflanzenextrakte, entzündungshemmende Wirkstoffe, die Wundheilung beschleunigende Stoffe (z.B. Chitin oder Chitosan und dessen Derivate), filmbildende Substanzen (z.B. Polyvinylpyrrolidone oder Chitosan oder dessen Derivate), Vitamine (z.B. Vitamin C und Derivate, Tocopherole und Derivate, Vitamin A und Derivate), 2-Hydroxycarbonsäuren (z.B. Citronensäure, Äpfelsäure, L-, D-, oder dl-Milchsäure), Hautaufhellungsmittel (z.B. Kojisäure, Hydrochinon oder Arbutin), Hautfärbungsmittel (z.B. Walnussextrakte oder Dihydroxyaceton), Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchthaltende Substanzen (z.B. Glycerin oder Harnstoff), Fette, Öle, ungesättigte Fettsäuren oder deren Derivate (z.B. Linolsäure, $\alpha$-Linolensäure, $\gamma$-Linolensäure oder Arachidonsäure und deren jeweiligen natürlichen oder synthetischen Ester), Wachse oder andere übliche Bestandteile einer kosmetischen oder dermatologischen Formulierung wie Alkohole, Polyole, Polymere, Schaumstabilisatoren, Elektrolyte, organische Lösungsmittel, Silikonderivate oder Chelatbildner (z.B. Ethylendiamintetraessigsäure und Derivate). Die jeweils einzusetzenden Mengen an kosmetischen oder dermatologischen Hilfs- und Zusatzstoffen und Parfüm können in Abhängigkeit von der Art des jeweiligen Produkts vom Fachmann durch einfaches Ausprobieren leicht ermittelt werden.

Besonders bevorzugt können die erfindungsgemäßen kosmetischen und/oder dermatologischen Zubereitungen, enthaltend erste und zweite Antioxidansmittel oder deren Salze, auch Wirkstoffe zur Hautaufhellung enthalten. Insbesondere können die erfindungsgemäßen topischen kosmetischen Mittel auch Benzaldoxime mit mindestens einer aromatischen Hydroxy- oder Alkoxygruppe, Kojisäure, Kojisäurederivate, Ascorbinsäure, Ascorbinsäurederivate, Hydrochinon, Hydrochinonderivate, schwefelhaltigen Moleküle (z.B. Glutathion oder Cystein) oder andere synthetische oder natürliche Wirkstoffe zur Hautaufhellung enthalten, wobei letztere auch in Form eines Extrakts aus Pflanzen (z.B. Tocopherole und Derivate, Arbutin (z.B. aus Bearberry-Extrakt), Aloesin (z.B. aus Aloe-Extrakt), Grapefruit-Extrakt und Reis-Extrakt) verwendet werden können.

Die Menge der vorgenannten beispielhaften Wirkstoffe zur Hautaufhellung (eine oder mehrere Verbindungen) kann in den erfindungsgemäßen kosmetischen oder dermatologischen Zubereitungen 0,001 bis 30 Gew.-%, bevorzugt 0,001 bis 20 Gew.-%, besonders bevorzugt 0,001 bis 5 Gew.-%, bezogen auf das Gesamtgewicht der Zubereitung, betragen.

Besonders bevorzugt sind solche kosmetischen oder dermatologischen Zubereitungen, die gleichzeitig in Form eines Sonnenschutzmittels vorliegen. Diese enthalten neben einem wirksamen Anteil zweiten Antioxidantien und ersten Antioxidantien auch Sonnenschutzsubstanzen, bevorzugt organische oder anorganische Lichtfiltersubstanzen, insbesondere Mikropigmente. Die erfindungsgemäßen Hautaufhellungsmittel können aber auch UVA- und/oder UVB-Filtersubstanzen enthalten, wobei die Gesamtmenge an Filtersubstanzen 0,1 bis 30 Gew.-%, vorzugsweise 0,5 bis 10 Gew.-%, bezogen auf das Gesamtgewicht der Zubereitungen, betragen kann, wobei man Sonnenschutzmitteln für Haut und Haar erhält. Als UV-Filtersubstanzen können beispielsweise 3-Benzylidencampherderivate (z.B. 3-(4-Methylbenzyliden)-dl-campher), Aminobenzoesäurederivate (z.B. 4-(N,N-Dimethylamino)benzoesäure-2-ethylhexylester oder Menthylanthranilat), 4-Methoxycinnamate (z.B. 2-Ethylhexyl-p-methoxycinnamat oder Isoamyl-p-methoxycinnamat), Benzophenone (z.B. 2-Hydroxy-4-methoxybenzophenon), ein- oder mehrfach sulfonierte UV-Filter [z.B. 2-Phenylbenzimidazol-5-sulfonsäure, Sulisobenzone oder 1,4-Bis(benzimidazolyl)-benzol-4,4',6,6'-tetrasulfonsäure bzw. 3,3'-(1,4-Phenylendimethyliden)-bis-(7,7-dimethyl-2-oxo-bicyclo-[2,2,1]heptan-1-methansulfonsäure) und deren Salze], Salicylate (z.B. 2-Ethylhexylsalicylat oder Homomenthylsalicylat), Triazine {z.B. 2,4-Bis-[4-(2-ethylhexyloxy)-2-hydroxyphenyl]-6-(4-methoxyphenyl)-1 ,3,5-triazin, 4,4'-([6-([(1,1-dimethylethyl)-aminocarbonyl]phenylamino)-1,3,5-triazin-2,4-diyl]diimino)bisbenzoesäurebis-(2-ethylhexyl)-ester)}, 2-Cyanopropensäurederivate (z.B. 2-Ethylhexyl-2-cyano-3,3-diphenyl-2-propenoat), Dibenzoylderivate (z.B. 4-tert-Butyl-4'-methoxydibenzoyl-methan), polymergebundende UV-Filter (z.B. Polymer von N-[2-(bzw. 4)-(2-Oxo-3-bornyliden)methyl]benzylacrylamid) oder Pigmente (z.B. Titandioxide, Zirkondioxide, Eisenoxide, Siliciumdioxide, Manganoxide, Aluminiumoxide, Ceroxide oder Zinkoxide) verwendet werden.

Die Lipidphase in den erfindungsgemäßen kosmetischen oder dermatologischen Zubereitungen, enthaltend erste und zweite Antioxidantien und/oder deren Salze, kann vorteilhaft gewählt werden aus folgenden Substanzgruppen: Mineralöle (vorteilhaft Paraffinöl), Mineralwachse, Kohlenwasserstoffe (vorteilhaft Squalan oder Squalen), synthetische oder halbsynthetische Triglyceridöle (z.B. Triglyceride der Caprin- oder Caprylsäure), natürliche Öle (z.B. Rizinusöl, Olivenöl, Sonnenblumenöl, Sojaöl, Erdnussöl, Rapsöl, Mandelöl, Palmöl, Kokusöl, Palmkernöl, Borretschsamenöl und dergleichen mehr), natürliche Esteröle (z.B. Jojobaöl), synthetische Esteröle (bevorzugt Ester von gesättigten und/oder ungesättigten, linearen und/oder verzweigten Alkancarbonsäuren von 3 bis 30 C-Atomen mit gesättigten und/oder ungesättigten, linearen und/oder verzweigten Alkoholen mit 3 bis 30 C-Atomen und Ester von aromatischen Carbonsäuren mit gesättigten und/oder ungesättigten, linearen und/oder verzweigten Alkoholen mit 3 bis 30 C-Atomen, insbesondere ausgewählt aus der Gruppe Isopropylmyristat, Isopropylstearat, Isopropylpalmitat, Isopropyloleat, n-Butylstearat, n-Hexyllaurat, n-Decyllaurat, Isooctylstearat, Isononylstearat, Isononylisononanoat, 2-Ethylhexylpalmitat, 2-Ethylhexyllaureat, 2-Hexyldecylstearat, 2-Octyldecylpalmitat, Oleyloleat, Oleylerucat, Erucyloleat, Erucylerucat sowie synthetische oder natürliche Gemische solcher Ester), Fette, Wachse und andere natürliche und synthetische Fettkörper, vorzugsweise Ester von Fettalkoholen mit Alkoholen niedriger C-Zahl (z.B. mit Isopropanol, Propylenglycol oder Glycerin) oder Ester von Fettalkoholen mit Alkansäuren niedriger C-Zahl oder mit Fettsäuren, Alkylbenzoate (z.B. Gemische von n-Dodecyl-, n-Tridecyl-, n-Tetradecyl- und n-Pentadecylbenzoat) sowie cyclische oder lineare Silikonöle (wie z.B. Dimethylpolysiloxane, Diethylpolysiloxane, Diphenylpolysiloxane sowie Mischformen daraus).

Die wässrige Phase der erfindungsgemäßen kosmetischen oder dermatologischen Zubereitungen, enthaltend erste und zweite Antioxidantien und/ oder deren Salze, enthält gegebenenfalls vorteilhaft Alkohole, Diole oder Polyole niedriger C-Zahl, sowie deren Ether, vorzugsweise Ethanol, Isopropanol, Propylenglycol, Glycerin, Ethylenglycol, Ethylenglycolmonoethyl- oder -monobutylether, Propylenglycolmonomethylether-, -monoethyl- oder -monobutylether, Diethylenglycolmonomethyl- oder monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft ausgewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, jeweils einzeln oder in Kombination oder aus der Gruppe der Polyurethane.

Insbesondere bevorzugt ist die Verwendung der erfindungsgemäßen Zubereitungen zum Schutz von Geweben und Zellen von Säugern vor potentiell allergenen Duftstoffen. Die erfindungsgemäßen kosmetischen oder dermatologischen Zubereitungen dienen vor allem dem Schutz der Haut.

Die Erfindung wird nachfolgend anhand von Ausführungsbeispielen weiter beschrieben.

Die Aktivität des Fulleren C60 als Antioxidans wurde mit der des herkömmlichen Antioxidans Tocopherol verglichen. Als Testsystem wurde die beschleunigte Autoxidation von Lipiden durch Luft mit oder ohne Antioxidant mit Hilfe einer Rancimat-Apparatur angewendet (Metrohm AG, Herisau, Schweiz).

α-Tocopherol und das Fulleren $C_{60}$ wurden in Methanol oder Aceton gelöst und von der jeweiligen Testlösung 100 ml zu einer vorbereiteten Ölprobe (Sojaöl, über Alumina Typ N gereinigt) von 3 g gegeben. In eine Kontrollprobe wurde nur Lösungsmittel gegeben. Durch die auf 100 °C aufgeheizte, die Testlösung enthaltende Ölprobe wurde ein konstanter, trockener Luftstrom (20 l/h) geblasen und die flüchtigen Oxidationsprodukte (vorwiegend kurzkettige Fettsäuren wie Ameisen- oder Essigsäure) in einer Vorlage mit Wasser gesammelt. Die Leitfähigkeit dieser wässrigen Lösung wurde kontinuierlich gemessen und dokumentiert. Die Oxidation von (ungesättigten) Fetten verläuft dabei eine Zeitlang nur sehr langsam und steigt dann plötzlich stark an. Die Zeit bis zum Anstieg wird als Induktionsperiode (IP) bezeichnet.

Nach der folgenden Gleichung wurde der antioxidative Index (AOI) erhalten:

$$AOI = IP(\text{mit Testlösung}) / IP(\text{Kontrollprobe})$$

Die Ergebnisse sind nachfolgend für die Riechstoffe Geraniol und Farnesol dargestellt:

| Antioxidans | Riechstoff | AOI |
|---|---|---|
| α-Tocopherol | Geraniol | 3,6 |

Tabelle fortgesetzt

| Antioxidans | Riechstoff | AOI |
|---|---|---|
| α-Tocopherol | Farnesol | 3,17 |
| Fulleren $C_{60}$ | Geraniol | 2,65 |
| Fulleren $C_{60}$ | Farnesol | 2,47 |

Das Antioxidans Fulleren $C_{60}$ hat daher eine dem α-Tocopherol vergleichbare antioxidative Wirkung und stabilisiert die Riechstoffe Geraniol und Farnesol vorteilhaft gegen Oxidation an Luft. Dadurch wird die allergene Wirkung dieser Riechstoffe vorteilhaft herabgesetzt und die Lagerungsstabilität dieser Riechstoffe verbessert.

## Patentansprüche

1. Riechstoffzubereitung, umfassend einen Riechstoff und ein erstes Antioxidans, **dadurch gekennzeichnet, dass** die Riechstoffzubereitung ferner ein zweites Antioxidans umfasst, wobei das zweite Antioxidans einen Fulleren-Körper besitzt.

2. Riechstoffzubereitung nach Anspruch 1, **dadurch gekennzeichnet, dass** das zweite Antioxidans in einem Anteil von 0,001 bis 3 Gew.-% bezogen auf die gesamte Riechstoffzubereitung vorliegt.

3. Riechstoffzubereitung nach einem der vorherigen Ansprüche, **dadurch gekennzeichnet, dass** der Fulleren-Körper des zweiten Antioxidans ein geschlossener Käfig-Körper ist.

4. Riechstoffzubereitung nach einem der vorherigen Ansprüche, wobei der Riechstoff bei Oxidation an Luft eine α, β-ungesättigte Carbonylfunktion bildet.

5. Kosmetisches und/oder dermatologisches Produkt, enthaltend eine Riechstoffzubereitung nach einem der vorherigen Ansprüche.

6. Verfahren zum Herstellen einer Riechstoffzubereitung, umfassend das Mischen eines Riechstoffs mit einem ersten und einem zweiten Antioxidans, wobei das zweite Antioxidans einen Fulleren-Körper besitzt.

7. Verwendung eines Antioxidans mit einem Fulleren-Körper zum Herstellen einer Riechstoffzubereitung mit verringerter allergener Wirkung.

8. Verwendung eines Antioxidans mit einem Fulleren-Körper zum Stabilisieren eines Riechstoffs gegen Oxidation.

9. Verwendung eines Antioxidans mit einem Fulleren-Körper zum Verbessern der Lagerungsstabilität eines Riechstoffs, einer Riechstoffzubereitung, eines kosmetischen und/oder dermatologischen Produkts.

Europäisches Patentamt

# EUROPÄISCHER RECHERCHENBERICHT

Nummer der Anmeldung
EP 04 02 7198

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| A | EP 0 520 700 A (WM. WRIGLEY JR. COMPANY)<br>30. Dezember 1992 (1992-12-30)<br>* Anspruch 1 * | 1-3,6,8,9 | C11B9/00<br>A61K7/00<br>A61K7/48 |
| D,A | EP 0 770 577 A (CHIANG, LONG Y)<br>2. Mai 1997 (1997-05-02)<br>* Seite 4, Zeile 46 - Zeile 48 * | 1-3,5 | |
| A | US 5 612 021 A (MELLUL ET AL)<br>18. März 1997 (1997-03-18)<br>* Beispiel 6 * | 1-3,5 | |

RECHERCHIERTE SACHGEBIETE (Int.Cl.7)

C11B
A61K
C01B

Der vorliegende Recherchenbericht wurde für alle Patentansprüche erstellt

| Recherchenort | Abschlußdatum der Recherche | Prüfer |
|---|---|---|
| Den Haag | 25. Mai 2005 | Saunders, T |

1

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 04 02 7198

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

25-05-2005

| Im Recherchenbericht angeführtes Patentdokument | Datum der Veröffentlichung | Mitglied(er) der Patentfamilie | Datum der Veröffentlichung |
|---|---|---|---|
| EP 0520700 A | 30-12-1992 | US 5139796 A | 18-08-1992 |
| | | AU 642599 B2 | 21-10-1993 |
| | | AU 1846992 A | 07-01-1993 |
| | | CA 2072447 A1 | 29-12-1992 |
| | | CN 1070315 A ,C | 31-03-1993 |
| | | DE 69205343 D1 | 16-11-1995 |
| | | DE 69205343 T2 | 09-05-1996 |
| | | DK 520700 T3 | 27-11-1995 |
| | | EP 0520700 A1 | 30-12-1992 |
| | | ES 2077980 T3 | 01-12-1995 |
| | | FI 922973 A | 29-12-1992 |
| | | JP 2588668 B2 | 05-03-1997 |
| | | JP 6184590 A | 05-07-1994 |
| | | MX 9203450 A1 | 01-12-1993 |
| | | PH 30667 A | 16-09-1997 |
| | | US 5200214 A | 06-04-1993 |
| EP 0770577 A | 02-05-1997 | US 5648523 A | 15-07-1997 |
| | | DE 69607437 D1 | 04-05-2000 |
| | | DE 69607437 T2 | 10-08-2000 |
| | | EP 0770577 A1 | 02-05-1997 |
| | | ES 2144204 T3 | 01-06-2000 |
| | | JP 9136964 A | 27-05-1997 |
| | | US 2003009036 A1 | 09-01-2003 |
| | | US 2003013861 A1 | 16-01-2003 |
| | | US 6020523 A | 01-02-2000 |
| | | US 2004034205 A1 | 19-02-2004 |
| | | US 6455709 B1 | 24-09-2002 |
| | | US 6380434 B1 | 30-04-2002 |
| | | US 5994410 A | 30-11-1999 |
| | | US 6046361 A | 04-04-2000 |
| US 5612021 A | 18-03-1997 | FR 2698002 A1 | 20-05-1994 |
| | | CA 2102932 A1 | 14-05-1994 |
| | | DE 69302886 D1 | 04-07-1996 |
| | | EP 0599687 A1 | 01-06-1994 |
| | | ES 2090921 T3 | 16-10-1996 |
| | | JP 2524476 B2 | 14-08-1996 |
| | | JP 6192039 A | 12-07-1994 |

EPO FORM P0461

Für nähere Einzelheiten zu diesem Anhang : siehe Amtsblatt des Europäischen Patentamts, Nr.12/82